# EUROPÄISCHE PATENTSCHRIFT

(11) **EP 2 468 315 B1**
(45) Veröffentlichungstag und Bekanntmachung des Hinweises auf die Patenterteilung: **07.02.2018**
(21) Anmeldenummer: 11009580.9
(22) Anmeldetag: 05.12.2011
(51) Int. Cl.: A61L 27/28, A61L 31/08, A61L 31/16, B05D 1/28

(54) **Beschichtungsverfahren und Beschichtungsvorrichtung**
Surface coating method and coating device
Procédé de revêtement et dispositif de revêtement

(30) Priorität: 23.12.2010 DE 102010055559
(43) Veröffentlichungstag der Anmeldung: 27.06.2012
(73) Patentinhaber: Heraeus Medical GmbH, 61273 Wehrheim (DE)
(72) Erfinder: Vogt, Sebastian, 99092 Erfurt (DE); Kühn, Klaus-Dieter, 35041 Marburg-Elnhausen (DE)
(74) Vertreter: Heraeus IP

(56) Entgegenhaltungen:
- WO-A1-2005/042045
- WO-A2-2007/050565
- US-A1- 2004 062 592
- US-A1- 2005 074 544
- US-B1- 7 563 324

## Beschreibung

Die Erfindung betrifft ein Verfahren zur zumindest bereichsweisen Beschichtung eines medizinischen Implantats, vorzugsweise eines künstlichen Gelenks oder einer Fixierung für ein Gelenk.

Die Erfindung betrifft auch eine Vorrichtung zur zumindest bereichsweisen Beschichtung eines medizinischen Implantats mit einem solchen Verfahren.

Die Beschichtung von medizinischen Implantaten mit pharmazeutischen Wirkstoffen hat in den letzten Jahren zunehmend Beachtung gefunden. Eine Hauptanwendung von Beschichtungsverfahren liegt dabei im antibiotischen Schutz der Oberfläche von Implantatmaterialien.

Die Implantation von Gelenkendoprothesen und auch von Osteosynthesematerialien ist immer mit einer gewissen Gefahr einer mikrobiellen Kontamination verbunden. Wenn es mikrobiellen Keimen gelingt, sich an der Implantatoberfläche anzusiedeln, kann es zur Ausbildung einer post-operativen Osteitis/Osteomyelitis kommen. Die Osteitis/Osteomyelitis stellt eine schwerwiegende Komplikation für den Patienten dar, die zusätzlich mit erheblichen Kosten verbunden ist.

Seit Jahrzehenten wird mit klinischem Erfolg bei zementierten Gelenkendoprothesen mit Gentamicin dotierter PMMA-Knochenzement verwendet. Das im Knochenzement enthaltende Breitbandantibiotikum Gentamicin schützt die Oberfläche des Knochenzementes wirksam gegen bakterielle Infektionen.

Bei nicht-zementierten Gelenkendoprothesen und bei Osteosynthesematerialien wurde eine Reihe von Lösungswegen vorgeschlagen, um ebenfalls einen lokalen antibiotischen Schutz der Implantatoberflächen zu erreichen.

So wurde in mehreren Patentdokumenten die Verwendung von in Wasser gering löslichen Antibiotika-Salzen beschrieben. Exemplarisch seien dafür die EP 0 623 349 A1, EP 1 470 829 A1, EP 1 374 923 A2, DE 101 42 465 A1 und DE 44 04 018 A1 genannt. Diese in Wasser gering löslichen Salze lösen sich unter Freisetzung der enthaltenen Antibiotika durch Einwirkung von Körperflüssigkeiten auf. Vorteilhaft ist die protrahierte Wirkstofffreisetzung. Nachteilig ist jedoch die aufwendige Herstellung dieser Salze.

US2005074544 offenbart ein Verfahren zur Beschichtung von medizinischen Implantaten (99) in einem Behälter (93). Das Implantat wird in den Behälter eingeführt und in Kontakt mit einem Übertragungsschwamm (97) als Mantel gebracht. Der Schwamm (97) wird mittels Rollen (92) und elastischen Armen (102) gegen das Implantat (99) gepresst.

Alternativ dazu ist es auch möglich, in Wasser lösliche Antibiotika-Salze zu verwenden. Das Problem liegt dabei darin, das Antibiotikum auf der Implantatoberfläche zu fixieren.

Die Mehrzahl der bisherigen beschriebenen Beschichtungen ist vorzugsweise für die Fertigung von beschichteten Implantaten unter industriellen Bedingungen bestimmt. Das bedeutet, dass die industrielle Beschichtung dieser Implantate nur mit wenigen für die Großanwendung relevanten Wirkstoffen erfolgen kann, um eine wirtschaftliche industrielle Fertigung mit entsprechenden hohen Stückzahlen gewährleisten zu können.

Insbesondere bei antibiotischen Beschichtungen ist es jedoch im Hinblick auf die sich zunehmend problematisch gestaltende Resistenzsituation und dem dabei vermehrten Auftreten von multiresistenten Keimen, wie MRSA und MRSE, von Interesse, genau auf die jeweiligen Keime abgestimmte Antibiotika oder Antibiotika-Kombinationen zur Beschichtung von Revisionsprothesen im Zuge des einzeitigen oder zweizeitigen septischen Gelenkprothesenwechsels einzusetzen, um einen initialen antibiotischen Schutz der Implantatoberflächen zu gewährleisten.

Nachteilig ist hieran, dass die Verfahren zur Beschichtung der medizinischen Implantate relativ aufwendig sind. Eine variable kurzfristige Anwendung ist nicht möglich. Es müssen für verschiedene Situationen verschiedene beschichtete medizinische Implantate bereitgehalten werden, um den Bedürfnissen der unterschiedlichen Patienten gerecht zu werden. Dies erfordert eine umfangreiche Lagerhaltung und verhindert ungewöhnliche Lösungen für spezielle Fälle.

Die Aufgabe der Erfindung besteht also darin, die Nachteile des Stands der Technik zu überwinden. Insbesondere soll ein einfaches und leicht anzuwendendes Verfahren und eine Vorrichtung hierfür bereitgestellt werden, mit denen eine medizinische Prothese beschichtet werden kann, ohne dass dadurch der Ablauf einer Operation (OP) gestört wird. Es sollen möglichst viele verschiedene medizinische Implantate mit dem Verfahren und der Vorrichtung beschichtet werden können. Auch sollen das Verfahren und die Vorrichtung variabel einsetzbar sein, so dass sie an die medizinischen Notwendigkeiten, insbesondere an eine für den Patienten geeignete Medikamentierung, anpassbar sind. Der in Operationssälen gebotenen Reinheit soll ebenfalls Rechnung getragen werden.

Die Aufgabe der Erfindung besteht ferner darin, ein möglichst einfaches Beschichtungsverfahren zu entwickeln, das vom OP-Personal während einer OP mit geringstem Zeitaufwand zum Beschichten von unterschiedlichsten Implantaten, die von beliebigen Herstellern produziert sein können, mit pharmazeutischen Zubereitungen eingesetzt werden kann. Weiterhin ist es eine Aufgabe der Erfindung, eine einfache Beschichtungsvorrichtung zu entwickeln, die es dem OP-Personal erlaubt, unter OP-Bedingungen Implantate mit geringstem Aufwand unter Verwendung beliebiger flüssiger pharmazeutischer Zubereitungen zu beschichten. Die Vorrichtung soll weiterhin so gestaltet sein, dass möglichst keine Sprühnebel oder Tropfen den OP-Bereich kontaminieren können. Eine weitere Aufgabe besteht darin, dass die Vorrichtung besonders zur Beschichtung von nicht-zementierten Gelenkendoprothesen und Osteosynthesematerialien geeignet sein soll.

Die Aufgaben der Erfindung werden durch das Verfahren nach Anspruch 1 gelöst. Erfindungsgemäße Verfahren erfolgen vor dem Einsetzen der medizinischen Implantate. Die Verfahren finden also "ex vivo" statt.

Unter einer pharmazeutisch aktiven Substanz sind erfindungsgemäß pharmazeutisch wirksame Mittel und Mittel zu verstehen, die pharmakologisch wirken, sowie solche, die eine pharmakologische Wirkung unterstützen oder sonst in irgendeiner Weise die Selbstheilungskräfte des Körpers unterstützen. Beispiele sind hierfür Antibiotika, organische Antiseptika, Kupfersalze, Kupferoxid, Galliumsalze, Strontiumsalze, Lithiumsalze, Silbersalze, Silberoxid, Bisphosphonate, Wachstumsfaktoren, Steroidhormone, nichtsteroidale Hormone, Hämostyptika, Antiphlogistika, Plasmide, Cosmide, lineare DNA und deren Mischungen.

Erfindungsgemäß kann auch vorgesehen sein, dass die Flüssigkeit eine wässrige Lösung eines Antibiotikums umfasst, bevorzugt eine wässrige Gentamicinsulfat-Lösung mit einem Gentamicinsulfat-Gehalt von 10,0 bis 88,0 Gewichtsprozent verwendet wird, wobei ganz besonders eine Gentamicinsulfat-Lösung mit einem Gentamicinsulfat-Gehalt von 75,0 bis 80,0 Gewichtsprozent bevorzugt verwendet wird. Diese Gentamicinsulfat-Lösung hat eine ölig-viskose Konsistenz und haftet sehr gut auf Metalloberflächen.

Es kann dabei ferner vorgesehen sein, dass pharmazeutisch übliche Stabilisatoren in den Gentamicinsulfat-Lösungen enthalten sind. Diese verbessern die Haltbarkeit und damit die Verwendbarkeit der aufzutragenden Flüssigkeit.

Erfindungsgemäß kann auch die Verwendung von anderen Aminoglykosid-Antibiotika-Lösungen, wie wässrige Lösungen des Tobramycinsulfats, des Amikacinsulfats, des Netilmicinsulfats und des Sisomycinsulfats als Flüssigkeit oder Flüssigkeitsbestandteile vorgesehen sein. Es ist auch möglich, wässrige Lösungen des Vancomycins, des Dalbavancins, des Ramoplanins, des Daptomycins, des Moxifloxacins, des Clindamycins und des Lincomycins einzusetzen.

Ferner kann im Rahmen der Erfindung die Verwendung von Kombinationen von Lösungen verschiedener Antibiotika als Flüssigkeit vorgesehen sein. Beispielhaft sind hierbei die Zweifachkombinationen von Gentamcinsulfat mit Vancomycinhydrochlorid, die Zweifachkombination von Daptomycin mit Gentamicinsulfat und die Zweifachkombination von Gentamicinsulfat mit Clindamycin sowie die Dreifachkombination Gentamicinsulfat mit Vancomycinhydrochlorid und Clindamycinhydrochlorid.

Des Weiteren kann vorgesehen sein, dass als Flüssigkeit Antiseptika-Lösungen verwendet werden, insbesondere Lösungen des Chlorhexidindigluconats, des Octenidindihydrochlorids und des Polyhexanids.

Erfindungsgemäß kann auch vorgesehen sein, dass die Flüssigkeit Lösungen von Antibiotika und Antiseptika umfasst, die als. Lösungsmittel organische Lösungsmittel oder Kombinationen von organischen Lösungsmitteln oder auch Kombinationen von organischen Lösungsmitteln und Wasser enthalten. Es ist dadurch möglich, zum Beispiel auch in Wasser gering lösliche Antibiotika-Salze, wie Laurate, Myristate, Palmitate und Stearate zu verwenden. Daneben können auch in Wasser gering lösliche Antibiotika oder Antibiotika-Salze in Form von wässrigen Suspensionen eingesetzt werden. Das erfindungsgemäße Verfahren sieht vor, dass die Flüssigkeit aus dem Übertragungsmittel heraus auf die zu beschichtende Oberfläche des medizinischen Implantats übertragen wird, vorzugsweise aus Kanälen, zumindest einer Vorratskammer, Poren, Fasern und/oder Zwischenräumen des Übertragungsmittels.

Ferner ist es von besonderem Vorteil, wenn vorgesehen ist, dass die Flüssigkeit in das Übertragungsmittel eingebracht wird, vorzugsweise in Kanäle, zumindest eine Vorratskammer, Poren und/oder Zwischenräume des Übertragungsmittels, oder das Übertragungsmittel mit der Flüssigkeit getränkt wird, insbesondere unmittelbar vor der Verwendung. Dadurch wird die Variabilität des Verfahrens sichergestellt.

Dabei kann vorgesehen sein, dass eine zur Behandlungssituation passende Flüssigkeit, insbesondere ein zur Behandlungssituation passendes Antibiotikum oder Antibiotika-Gemisch in das Übertragungsmittel eingebracht wird. Eine an die speziellen Erfordernisse eines bestimmten Patienten angepasste Lösung kann somit kurz vor dem eigentlichen Beschichten des medizinischen Implantats bereitgestellt werden.

Zur Verhinderung einer Verunreinigung der Umgebung mit der Flüssigkeit ist es vorgesehen, dass das medizinische Implantat vor dem Kontakt mit dem Übertragungsmittel in einen Behälter eingeführt wird, in dem sich das Übertragungsmittel befindet und nach der Übertragung der Flüssigkeit auf das medizinische Implantat aus dem Behälter herausgezogen wird.

Eine weitere Verbesserung kann dadurch erreicht werden, dass nach dem Übertragen der Flüssigkeit auf das medizinische Implantat ein Pulver auf die benetzte Oberfläche des medizinischen Implantats aufgebracht wird, bevorzugt das medizinische Implantat in ein Pulver eingetaucht wird. Dieses Pulver kann beispielsweise zumindest eine pharmazeutisch aktive Substanz oder zumindest eine knochenwachstumsstimulierende Verbindung umfassen. Ein geeignetes Pulver kann beispielsweise das Knochenwachstum fördern und so den Erfolg einer Implantation verbessern, da das Pulver eine stabilere Verbindung des Implantats mit dem angrenzenden Knochenmaterial bewirkt.

Auch kann vorgesehen sein, dass das medizinische Implantat durch zumindest eine Membran gestoßen wird oder zumindest eine Membran geöffnet wird, bevor das medizinische Implantat mit dem elastisch deformierbaren Übertragungsmittel kontaktiert wird, beispielsweise auf das elastisch deformierbare Übertragungsmittel gedrückt und/oder über das elastisch deformierbare Übertragungsmittel gestrichen wird, in das Pulver getaucht und/oder auf das Pulver gedrückt wird, wobei die zumindest eine Membran die Flüssigkeit und/oder das Pulver zumindest bereichsweise abdeckt, vorzugsweise die zumindest eine Membran das Pulver im Behälter abdichtet. Die Membran verhindert eine Kontamination der Flüssigkeit und/oder des Pulvers vor der Anwendung. Durch das Durchstoßen der Membran wird sichergestellt, dass die schützende Membran erst kurz vor der Anwendung geöffnet wird. Die Membran sollte dazu derart aufgebaut sein, dass keine Fetzen oder andere Teile der Membran in die Flüssigkeit beziehungsweise das Pulver gelangen können oder am medizinischen Implantat haften.

Eine weitere Ausgestaltung des erfindungsgemäßen Verfahrens kann vorsehen, dass ein zur Behandlungssituation passendes Pulver bereitgestellt wird.

Auch kann vorgesehen sein, dass ein zur Behandlungssituation passendes Antibiotikum oder Antibiotika-Gemisch in das Pulver eingebracht wird. Durch diese beiden Maßnahmen kann auf die konkrete Behandlungssituation beim jeweiligen Patienten eingegangen werden.

Besonders vorteilhafte Ausgestaltungen der Erfindung zeichnen sich dadurch aus, dass das Pulver als knochenwachstumsfördernde Substanz Calciumphosphat-Pulver, besonders bevorzugt ein Gemisch aus α- und β-Calciumphosphat, umfasst. Eine Reduzierung der Gefahr einer möglichen Verunreinigung der Umgebung und eine Einsparung der oft teuren Materialien zur Beschichtung lässt sich erfindungsgemäß dadurch erreichen, dass ein Teil der übertragenen Flüssigkeit und/oder des Pulvers abgestreift wird, insbesondere beim Herausziehen des medizinischen Implantats aus dem Behälter, vorzugsweise an einem dafür vorgesehenen Abstreifer. Hierdurch kann eine Kontamination der Umgebung, also insbesondere eines OP-Bereichs, mit der Flüssigkeit und gegebenenfalls auch mit dem Pulver verhindert oder zumindest reduziert werden. Dies ist vor allem bei der Verwendung von Antibiotika geboten, da so der Entwicklung resistenter Keime im OP-Bereich vorgebeugt werden kann.

Um den beschichteten Bereich und die Vollständigkeit der Beschichtung sichtbar zu machen, kann erfindungsgemäß vorgesehen sein, dass die Flüssigkeit eingefärbt wird, so dass der beschichtete Bereich des medizinischen Implantats farblich kenntlich gemacht wird.

Dabei kann vorgesehen sein, dass die Vollständigkeit der Beschichtung des zu beschichtenden Bereichs anhand der Färbung geprüft wird.

Erfindungsgemäß kann auch vorgesehen sein, dass das zu beschichtende Implantat mehrfach mit dem elastisch deformierbaren Übertragungsmittel kontaktiert wird, vorzugsweise mehrfach auf das elastisch deformierbare Übertragungsmittel gedrückt wird und/oder über das elastisch deformierbare Übertragungsmittel gestrichen wird.

Es kann ferner vorgesehen sein, dass das Verfahren so oft wiederholt wird, bis eine vollständige Beschichtung der zu beschichtenden Oberfläche des medizinischen Implantats erreicht wird. Insbesondere im Zusammenhang mit einer Färbung der Flüssigkeit und der Prüfung der Vollständigkeit der Beschichtung mit Hilfe der Färbung ist dies erfindungsgemäß vorteilhaft, um eine ausreichend beschichtetes medizinisches Implantat zu erhalten.

Auch kann erfindungsgemäß vorgesehen sein, dass zumindest 50% der Oberfläche des medizinischen Implantats, vorzugsweise zumindest 80%, besonders bevorzugt 90% der Oberfläche des medizinischen Implantats beschichtet werden.

Eine besonders vorteilhafte Ausgestaltung des Verfahrens ist dadurch gekennzeichnet, dass als medizinisches Implantat nicht zu zementierende Hüftgelenkendoprothesen, Schultergelenkendoprothesen, Ellenbogenprothesen, Marknägel oder Osteosyntheseplatten verwendet werden.

Bezüglich der Vorrichtung werden die Aufgaben durch die Vorrichtung nach Anspruch 9 gelöst. Dabei ist es vorgesehen, dass das Übertragungsmittel in einem Behälter umfassend eine Öffnung zum Einführen und Herausnehmen des medizinischen Implantats angeordnet ist. Der Behälter dient dazu, ein ungewolltes Verspritzen der Flüssigkeit zu vermeiden.

Dabei kann wiederum vorgesehen sein, dass die Öffnung durch einen abziehbaren Deckel verschlossen ist. Dadurch können Verunreinigungen des Inhalts vor der Anwendung der Vorrichtung vermieden werden.

Erfindungsgemäß kann des Weiteren vorgesehen sein, dass die Vorrichtung einen Abstreifer umfasst, der vorzugsweise im Bereich der Öffnung, insbesondere zwischen der Öffnung und dem Übertragungsmittel; angeordnet ist.

Dabei kann vorgesehen sein, dass der Abstreifer scheibenförmig ist und mindestens einen Einschnitt umfasst, der die Oberseite und die Unterseite der Scheibe verbindet.

Alternativ kann vorgesehen sein, dass der Abstreifer als Kegelmantel oder als Halbkugelfläche ausgebildet ist, wobei die Kegelspitze oder die Halbkugel in Richtung des Übertragungsmittels ausgerichtet ist und vorzugsweise der Kegel oder die Halbkugel mindestens einen Einschnitt enthalten, der die Oberseite und die Unterseite des Abstreifers verbindet. Dadurch können sich eventuell vom Übertragungsmittel während des Herausziehens des Implantats ablösende Tröpfchen und Partikel nur so fliegen, dass diese gegen die Innenwand des Behälters und gegen die Unterseite des Abstreifers prallen und somit eine Kontamination der Umgebung mit der Flüssigkeit vermindert wird.

Besonders vorteilhafte Vorrichtungen zeichnen sich dadurch aus, dass das Übertragungsmittel Poren umfasst und in den Poren des Übertragungsmittels die Flüssigkeit, vorzugsweise in Form einer Lösung und/oder Suspension, enthalten ist.

Eine weitere Ausgestaltung der Erfindung sieht vor, dass die pharmazeutisch aktive Substanz Antibiotika und/oder organische Antiseptika beinhaltet, so dass die zu erzeugende Beschichtung eine pharmazeutisch wirksame Dosis beinhaltet.

Auch kann vorgesehen sein, dass die Vorrichtung einen Vakuumanschluss umfasst, der mit einer Vakuumquelle verbindbar ist und der vorzugsweise zwischen dem Abstreifer und dem Übertragungsmittel angeordnet ist. Dadurch kann zusätzlich durch Absaugen von eventuellen Tröpfchen der Lösung und/oder Suspension sichergestellt werden, dass keine Kontamination der Umgebung mit pharmazeutischen Wirkstoffen erfolgt.

Gemäß einer weiteren Ausgestaltung der Erfindung kann vorgesehen sein, dass das Übertragungsmittel aus einem hydrophilen Material gefertigt ist und vorzugsweise zumindest ein anderes Teil, insbesondere der Behälter und/oder der Abstreifer, aus einem hydrophoben Material gefertigt sind. Bevorzugt werden wässrige Lösungen und/oder Suspensionen von pharmazeutischen Wirkstoffen für die Beschichtung verwendet. Wenn das Übertragungsmittel aus einem hydrophilen Material gefertigt ist, befinden sich wässrige Lösungen und/oder Suspensionen bevorzugt in dem porösen hydrophilen Material und nicht auf der hydrophoben Oberfläche des Behälters und des Abstreifers. Dieses Verhalten ermöglicht es, das mit wässrigen Lösungen und/oder Suspensionen vorgefüllte Beschichtungsvorrichtungen mit geringsten Volumina dieser wässrigen Lösungen oder Suspensionen auskommen und trotzdem eine sichere Beschichtung ermöglichen.

Ferner kann vorgesehen sein, dass der Abstreifer aus biokompatiblem Elastomer, thermoplastischem Kunststoff, aus einer Metallfolie oder aus Kompositen, die aus Metall-Elastomer-Kombinationen oder Metall-Kunststoffkombinationen gefertigt sind, aufgebaut ist.

Eine weitere Ausgestaltung der Erfindung sieht vor, dass der Abstreifer als Ring ausgebildet ist, der radial zum Mittelpunkt des Behälters angeordnete Borsten enthält, die gegebenenfalls einen Kegel bilden können, dessen Spitze in Richtung des Übertragungsmittels angeordnet ist. Diese Borsten können aus Kunststoff gebildet sein, wobei die Borsten vorzugsweise mechanisch so stabil sind und so stabil verankert sind, dass eine Ablösung oder ein Abbrechen der Borsten unterbleibt.

Auch kann vorgesehen sein, dass der Abstreifer als durch Walzen und/oder Kugeln ausgebildet ist, die durch elastische Verbindungsmittel mit dem Behälter verbunden sind. Erfindungsgemäß ist das Übertragungsmittel porös und elastisch und vorzugsweise aus einem porösen, hydrophilen Kunststoff gebildet ist.

Dabei ist es vorgesehen, dass das poröse, elastische Übertagungsmittel als Kegelmantel oder als plane Scheibe oder als gewölbte Scheibe ausgebildet ist, wobei mindestens ein Einschnitt die Oberseite des Übertragungsmittels mit der Unterseite des Übertragungsmittels verbindet und im Falle eines Kegelmantels die Kegelspitze in Richtung des Abstreifers angeordnet ist. Durch diesen mindesten einen Einschnitt kann das Implantat in die Vorrichtung eingebracht werden.

Besonders vorteilhaft ist es, wenn dabei vorgesehen ist, dass radiale Einschnitte in dem Abstreifer ausgebildet sind. Dann ist es möglich, dass der gesamte äußere Umfang der Implantate nach Beendigung der Beschichtung abgestreift wird und damit überschüssige Mengen der Lösung oder Suspension von der beschichteten Implantatoberfläche entfernt werden. Weiterhin ist es dadurch möglich, die Freisetzung von Tröpfchen oder Partikeln der Lösung oder Suspension, die beim Herausziehen des Implantats aus dem Übertragungsmittel entstehen können, wirksam zurückzuhalten. Damit wird die Kontamination der Umgebung weitgehend vermieden.

Ferner kann vorgesehen sein, dass die Vorrichtung mit einer wässrigen oder organischwässrigen oder organischen Lösung und/oder einer Suspension eines mindestens eines pharmazeutischen Wirkstoffs gefüllt ist oder zunächst keinen pharmazeutischen Wirkstoff enthält. Auch kann vorgesehen sein, dass der Abstreifer aus biokompatiblem Elastomer, thermoplastischem Kunststoff oder auch durch eine Metallfolie oder auch aus Kompositen, die aus Metall-Elastomer-Kombinationen oder Metall-Kunststoff-Kombinationen gefertigt sind, besteht. Erfindungsgemäß ist, dass als Pulver ß-Tricalciumphosphat, α-Tricalciumphosphat, amorphisiertes Calciumphosphat, Tetracalciumphosphat, Octacalciumphosphat, Hydroxylapatit, Fluorapatit, Calciumsulfat-Hemihydrat, Calciumsulfat-Dihydrat, wasserfreies Calciumsulfat, pulverförmige Antibiotika, organische Antiseptika, Kupfersalze, Kupferoxid, Galliumsalze, Strontiumsalze, Lithiumsalze, Silbersalze, Silberoxid, Bisphosphonate, Wachstumsfaktoren, Steroidhormone, nichtsteroidale Hormone, Hämostyptika, Antiphlogistika, Plasmide, Cosmide, lineare DNA sowie deren Gemische verwendet werden. In dem Pulver können auch Komplexbildner oder auch Salze enthalten sein, die mit den vom Abstreifer auf die Implantatoberfläche übertragenen pharmazeutischen Wirkstoffen in Wasser gering lösliche komplexe oder Salze bilden. So kann zum Beispiel Teicoplanin im Pulver enthalten sein, das mit Gentamicin oder anderen kationischen Antibiotika in Wasser gering lösliche Komplexe bildet. Es ist auch beispielsweise möglich, dass in dem Pulver N-Methyl-Glucamoniumsalze von Fettsäuren oder von Alkylsulfaten enthalten sind, die bei Kontakt mit wässrigen Lösungen von kationischen Antibiotika infolge eines reziproken Salzaustauschs in Wasser gering lösliche Fettsäuresalze oder Alkylsulfate der Antibiotika bilden können. Auf diese Weise ist es möglich, in Wasser gering lösliche Komplexe oder Salze von pharmazeutischen Wirkstoffen, insbesondere von Antibiotika, auf die Implantatoberfläche aufzubringen.

Besonders vorteilhaft ist es, wenn reaktive anorganische Pulver, wie amorphisiertes Calciumphosphat, Tetracalciumphosphat und Calciumsulfat-Hemihydrat verwendet werden, die in Gegenwart von Wasser aushärten. Dadurch ist es möglich, stabile Beschichtungen auszubilden. Die Aushärtung innerhalb von wenigen Sekunden kann dabei erreicht werden, indem zum Beispiel bei Verwendung von Calciumsulfat-Hemihydrat als Pulver geringe Mengen von Calciumsulfat-Dihydrat als Keimbildner sowie Ammoniumsulfat, Natriumsulfat oder Kaliumsulfat als Beschleuniger zu dem Calciumsulfat-Hemihydrat zugegeben werden. Vorteilhaft ist weiterhin die Verwendung von ß-Tricalciumphosphat, α-Tricalciumphosphat und Tetracalciumphosphat, die durch Einwirkung von wässrigen Säuren, insbesondere von wässrigen Lösungen der Äpfelsäure, der Weinsäure und der Citronensäure innerhalb von wenigen Sekunden aushärten.

Der Erfindung liegt die überraschende Erkenntnis zugrunde, dass durch die Verwendung von Übertragungsmitteln eine Beschichtung ermöglicht wird, die während einer OP oder kurz davor aufgetragen wird. Dadurch lassen sich die Beschichtungen an die individuellen Bedürfnisse der Patienten anpassen. Gleichzeitig wird durch die Verwendung der Übertragungsmittel das Verspritzen der Flüssigkeit vermindert und so eine Kontamination der Umgebung verhindert. Dies ist vor allem bei einer Anwendung in einem Operationssaal wichtig.

Damit wird ein erfindungsgemäßes Verfahren zur schnellen und unkomplizierten Beschichtung von medizinischen Implantaten mit pharmazeutischen Zubereitungen unter OP-Bedingungen bereitgestellt. Bei vielen erfindungsgemäßen Ausgestaltungen der Erfindung wird zudem erreicht, dass die Freisetzung von Wirkstofftröpfchen oder Wirkstoffspritzern in den OP-Bereich weitgehend vermieden wird. Die Beschichtungsvorrichtung ist besonders zur kostengünstigen Beschichtung von nicht-zementierten Gelenkendoprothesen, nicht-zementierten Revisions-Gelenkendoprothesen und von Osteosynthesematerialien mit pharmazeutischen Wirkstoffen vorgesehen.

Anstatt also das medizinische Implantat lange im Voraus bei der Herstellung zu beschichten, kann es auch unmittelbar vor dem Einsetzen beschichtet werden. Dadurch können auch relativ kurzlebige Beschichtungen verwendet werden. Zudem kann sogar eine noch flüssige Schicht verwendet werden, was neue Anwendungsgebiete eröffnet und neue Wirkstoffe zugänglich macht.

Die Vorrichtung kann mit einer Wirkstofflösung oder Wirkstoffsuspension vorbefüllt sein, so dass das OP-Personal nur die Vorrichtung öffnen muss und anschließend sofort die Beschichtung des Implantats vornehmen kann. Vorteilhaft ist hierbei, dass der Zeitaufwand für diese Beschichtung nur im Bereich von wenigen Sekunden liegt und wertvolle OP-Zeit gespart werden kann.

Alternativ ist es möglich, eine nicht befüllte Vorrichtung direkt im OP durch Einspritzen einer Wirkstofflösung oder Wirkstoffsuspension mit einem oder mehreren pharmazeutischen Wirkstoffen auszurüsten. Dadurch ist es im Fall der antibiotischen Beschichtung möglich, entsprechend der vorliegenden Resistenzsituation eine geeignete Auswahl eines Antibiotikums oder einer Antibiotika-Kombination vorzunehmen und damit eine Antibiogramm-gerechte Beschichtung herzustellen.

Es ist auch möglich, vor einer OP nicht befüllte Vorrichtungen in der jeweiligen Klinikapotheke mit geeigneten Wirkstofflösungen oder Wirkstoffsuspensionen zu befüllen, so dass während der OP die Beschichtung ohne Zeitverzug vorgenommen werden kann.

Besonders geeignet ist dabei, dass als pharmazeutisch aktive Substanz wenigstens ein Mitglied aus der Gruppe der pharmazeutischen Wirkstoffe, wie beispielsweise Antibiotika, organische Antiseptika, Kupfersalze, Kupferoxid, Galliumsalze, Strontiumsalze, Lithiumsalze, Silbersalze, Silberoxid, Bisphosphonate, Wachstumsfaktoren, Steroidhormone, nichtsteroidale Hormone, Hämostyptika, Antiphlogistika, Plasmide, Cosmide, lineare DNA und deren Mischungen vergewendet werden.

Für einen initialen antibiotischen Schutz reicht es aus, wenn für einen Zeitraum von 24 bis 72 Stunden hinreichend hohe Antibiotikum- oder Antibiotika-Konzentrationen an den Implantatoberflächen vorhanden sind. Daher kann schon bei der lokalen Einbringung von einfachen in Wasser löslichen Antibiotika ein ausreichender temporärer, lokaler, antibiotischer Schutz des medizinischen Implantats erzielt werden.

Die Vorrichtung kann als Arzneimittel oder als Medizinprodukt bereitgestellt werden.

Auch eine Kombination der erfindungsgemäßen Vorrichtung mit einem medizinischen Implantat könnte angeboten werden. Diese Kombination wird aus der Vorrichtung und dem Implantat gebildet, wobei diese Kombination eine Mindestlebensdauer von 0,1 Sekunden hat. Die Kombination entsteht besteht während des Beschichtungsprozesses.

Im Folgenden werden Ausführungsbeispiele der Erfindung anhand von fünf schematisch dargestellten Figuren erläutert, ohne jedoch dabei die Erfindung zu beschränken. Dabei zeigt:
- Figur 1:: eine schematische Querschnittansicht eines erfindungsgemäßen Übertragungsmittels in einem Behälter und
- Figur 2:: eine schematische perspektivische Ansicht einer erfindungsgemäßen Vorrichtung.

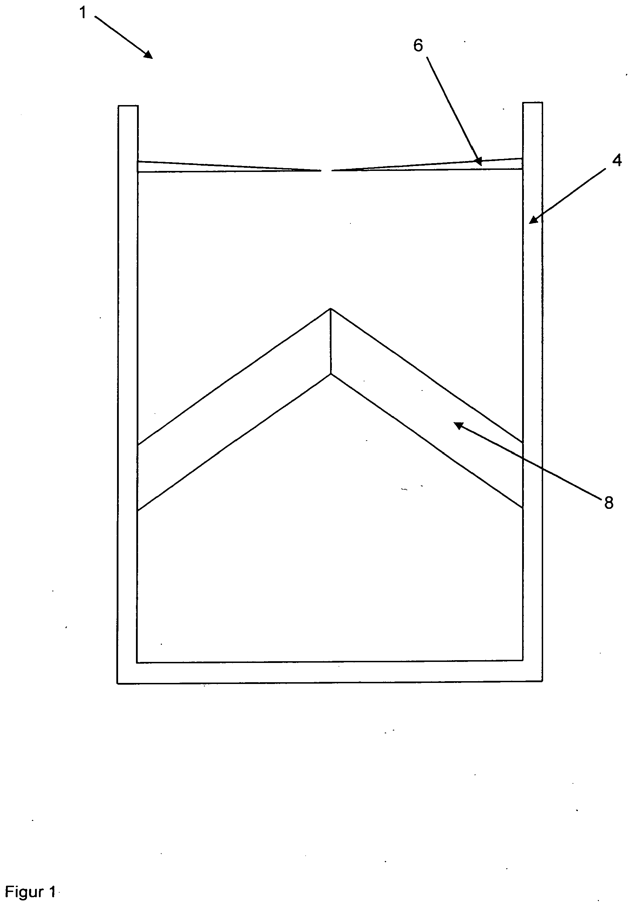

Figur 1 zeigt eine schematische Querschnittansicht einer erfindungsgemäßen Vorrichtung 1. Die Vorrichtung 1 umfasst einen Behälter 4 in Form eines oben offenen Topfs. Die Seitenwände des Behälters 4 sind zylindrisch und von gleichmäßiger Dicke. Im Bereich der Öffnung, kurz unterhalb der Öffnung, ist im Inneren des Behälters 4 ein Abstreifer 6 angeordnet, der die Öffnung fast vollständig, bis auf eine kreisrunde Öffnung in der Mitte abschließt.

Der Boden und die Seitenwände des Behälters 4 und der Abstreifer 6 sind aus einem hydrophoben Material gefertigt oder mit einer hydrophoben Schicht beschichtet. Ausgehend von der kreisrunden Öffnung des Abstreifers 6 ist der Abstreifer 6 in acht Richtungen geschlitzt beziehungsweise eingeschnitten.

Die acht Schlitze / Einschnitte (nicht gezeigt) reichen nicht bis zu den Seitenwänden des Behälters 4 und sollen das Einführen eines medizinischen Implantats durch den Abstreifer 6 ermöglichen. Der Abstreifer 6 hat dadurch acht flexible Segmente die beim Einführen und beim Herausnehmen, beziehungsweise beim Herausziehen des medizinischen Implantats, dieses Abstreifen, das heißt die Oberfläche des Implantats überstreichen. Der Durchmesser der kreisrunden Öffnung des Abstreifers 6 ist kleiner als der Querschnitt der einzuführenden medizinischen Implantate. Dadurch ist sichergestellt, dass der Abstreifer 6 im Wesentlichen die gesamte Oberfläche des medizinischen Implantats, insbesondere beim Herausziehen, überstreicht und dabei abstreift.

Im Inneren des Behälters 4 ist ein Übertragungsmittel 8 angeordnet, in dem ebenfalls Schlitze angeordnet sind. Das Übertragungsmittel 8 ist aus einem flexiblen porösen Material, wie einem Schwamm, gefertigt. Das Übertragungsmittel 8 ist mit einer wässrigen Lösung umfassend ein Antibiotikum vollgesogen. Das Material ist hydrophil. Dadurch wird sichergestellt, dass es mit einer wässrigen Flüssigkeit getränkt werden kann. Durch die hydrophoben Eigenschaften des Behälters 4 und des Abstreifers 6 befindet sich die wässrige Flüssigkeit vor allem im Übertragungsmittel 8.

Mit der gezeigten Vorrichtung 1 kann ein erfindungsgemäßes Verfahren durchgeführt werden. Das Übertragungsmittel 8 wird mit einer wässrigen Flüssigkeit getränkt, die zumindest eine pharmazeutisch wirksame Substanz enthält, mit der ein medizinisches Implantat beschichtet werden soll. Das Übertragungsmittel 8 kann über einen Anschluss (nicht gezeigt) mit der Flüssigkeit getränkt werden. Alternativ kann durch die kreisrunde Öffnung im Abstreifer 6 oder durch den Abstreifer 6 hindurch das Übertragungsmittel 8 mit einer Spritze befüllt werden.

Ein medizinisches Implantat (nicht gezeigt) wird durch den Abstreifer 6 hindurch geschoben und trifft auf das Übertragungsmittel 8. Durch den Druck, der über das medizinische Implantat auf das Übertragungsmittel 8 ausgeübt wird, schiebt sich zum einen das medizinische Implantat durch die dafür vorgesehenen Durchbrechungen im Übertragungsmittel 8 und zum anderen wird die in den Poren des Übertragungsmittels 8 enthaltene Flüssigkeit aus dem Übertragungsmittel 8 herausgedrückt und auf die Oberfläche des medizinischen Implantats aufgetragen.

Nachdem die Oberfläche des medizinischen Implantats beschichtet wurde, wird es aus dem Behälter 4 herausgezogen. Dabei wird die beschichtete Oberfläche des medizinischen am Abstreifer 6 vorbeigezogen. Die überschüssige Flüssigkeit wird von der Oberfläche des medizinischen Implantats abgestreift und tropft zurück auf das Übertragungsmittel 8. Das aus dem Behälter 4 herausgezogene medizinische Implantat tropft dann nicht mehr nach. Zudem wird durch die Neigung des Übertragungsmittels 8, das als Kegelmantel ausgeformt ist und dessen Spitze in Richtung des Abstreifers 6 weist, ein Herumspritzen der Flüssigkeit vermieden. Durch diese beiden Maßnahmen kann verhindert werden, dass die Flüssigkeit die Umgebung kontaminiert. Das mit der Flüssigkeit beschichtete medizinische Implantat ist dann bereit, um für eine Operation eingesetzt zu werden.

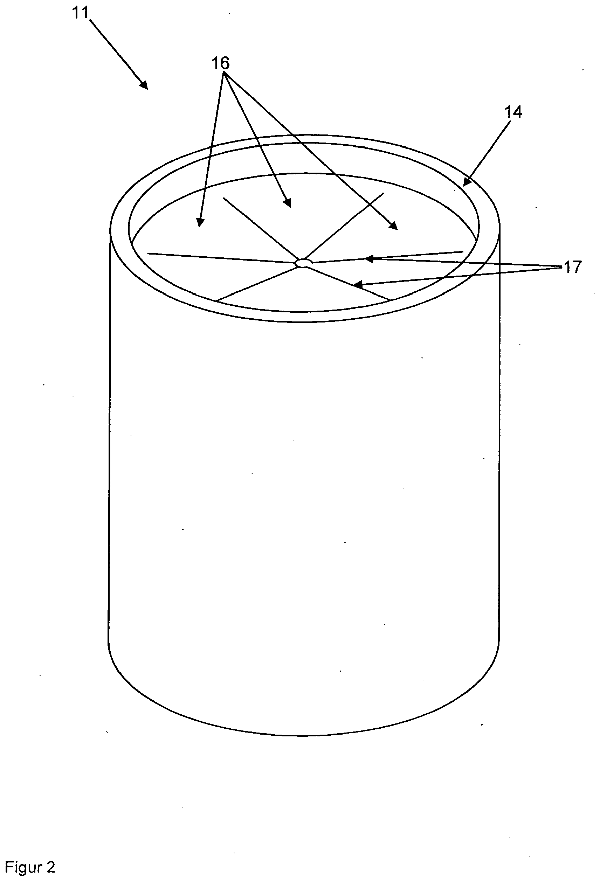

Figur 2 zeigt eine schematische perspektivische Ansicht einer zweiten erfindungsgemäßen Vorrichtung 11 für ein erfindungsgemäßes Verfahren. Die Vorrichtung 11 umfasst einen Behälter 14 und einen Abstreifer 16, der den Behälter 14 auf der Oberseite vollständig abdeckt. Der flexible Abstreifer 16 hat sechs Schlitze 17 beziehungsweise Einschnitte 17, die die Oberseite des Abstreifers 16 mit der ins Innere des Behälters 14 gewandten Unterseite des Abstreifers 16 verbinden, so dass ein medizinisches Implantat (nicht gezeigt) durch den Abstreifer 16 in das Innere des Behälters 14 entlang der dann umgeklappten Schlitze 17 eingeführt werden kann.

Im Inneren des Behälters 14 befindet sich ein Übertragungsmittel (nicht gezeigt), das aus drehbaren Walzen aufgebaut ist, auf deren Oberfläche eine Flüssigkeitsschicht ausgebildet ist, mit der das medizinische Implantat beschichtet werden kann. Ein Flüssigkeitsreservoir versorgt die Walzen mit Flüssigkeitsnachschub. Wenn ein medizinisches Implantat durch den Abstreifer 16 ins Innere des Behälters 14 geschoben wird, übertragen die Walzen des Übertragungsmittels die Flüssigkeit auf die Oberfläche des medizinischen Implantats und beschichten es dadurch.

Der Abstreifer 16 sorgt dafür, dass überschüssige Flüssigkeit von der Oberfläche des medizinischen Implantats abgestreift wird.

Im Folgenden werden Beispiele zur Herstellung einer Flüssigkeit für ein erfindungsgemäßes Verfahren und ein weiteres Beispiel für eine erfindungsgemäße Vorrichtung ausgeführt.

### Beispiel 1: Herstellung einer Beschichtungslösung mit Gentamicinsulfat

Es wurden 16,0 g Gentamicinsulfat (Fujian Fukang Ltd.) mit 4,0 ml pyrogenfreiem sterilem Wasser bei Raumtemperatur vermischt. Nach 24 Stunden Rühren bei Raumtemperatur mit einem Magnetrührer hatte sich eine ölig-viskose gelbliche Lösung gebildet. Es ergab sich eine Beschichtungslösung mit Gentamicinsulfat als Flüssigkeit zur Beschichtung eines medizinischen Implantats.

### Beispiel 2: Herstellung einer Beschichtungslösung mit der Zweifachkombination Gentamicinsulfat und Clindamycinhydrochlorid:

Es wurden 12,0 g Gentamicinsulfat (Fujian Fukang Ltd.), 4,0 g Clindamycinhydrochlorid (Sigma-Aaldrich) mit 4,0 ml pyrogenfreiem sterilem Wasser bei Raumtemperatur vermischt. Nach 24 Stunden Rühren bei Raumtemperatur mit einem Magnetrührer hatte sich eine ölig-viskose gelbliche Lösung gebildet.

### Beispiel 3: Herstellung einer Beschichtungslösung mit der Dreifachkombination Gentamicinsulfat, Clindamycinhydrochlorid und Vancomycinhydrochlorid:

Es wurden 4,0 g Gentamicinsulfat (Fujian Fukang Ltd.), 4,0 g Clindamycinhydrochlorid (Sigma-Aaldrich) und 4,0 g Vancomycinhydrochlorid (Sigma-Aldrich) mit 8,0 ml pyrogenfreiem sterilem Wasser bei Raumtemperatur vermischt. Nach 24 Stunden Rühren bei Raumtemperatur mit einem Magnetrührer hatte sich eine viskose gelbliche Lösung gebildet.

### Beispiel 4: Herstellung eines beschichteten Implantats

Mit konventionellen 10 ml Kunststoffspritzen wurden jeweils 5 ml der Beschichtungslösungen der aufgeführten Beispiele aufgezogen. Dann wurden mit den befüllten Kunststoffspritzen 4 ml der jeweiligen Wirkstofflösung auf ein poröses Übertragungsmittel einer erfindungsgemäßen Vorrichtung gespritzt. Die Wirkstofflösung wurde dabei von dem porösen Übertragungsmittel aufgesaugt.

Anschließend wurden übliche Zweymüller-Hüftprothesen kurz in eine befüllte erfindungsgemäße Vorrichtung kurz bis zum Ende des Schafts hineingetaucht und sofort wieder herausgezogen. Die Zweymüller-Hüftprothesen hatten dann einen zähflüssigen Film der Wirkstofflösung an der Schaftoberfläche.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 11: Vorrichtung
- 4, 14: Behälter
- 6, 16: Abstreifer
- 8: Übertragungsmittel
- 17: Schlitz / Einschnitt

## Patentansprüche

1. Verfahren zur zumindest bereichsweisen Beschichtung eines medizinischen Implantats, wobei
ein medizinisches Implantat mit einer zu beschichtenden Oberfläche bereitgestellt wird und
die zu beschichtende Oberfläche des medizinischen Implantats mit einem elastisch deformierbaren Übertragungsmittel, das wenigstens eine Flüssigkeit aufweist, die wenigstens eine pharmazeutisch aktive Substanz enthält, kontaktiert wird, wobei beim Kontaktieren des Übertragungsmittels die Flüssigkeit vom Übertragungsmittel auf die zu beschichtende Oberfläche des medizinischen Implantats übertragen wird,
**dadurch gekennzeichnet, dass**
das medizinische Implantat vor dem Kontakt mit dem Übertragungsmittel in einen Behälter eingeführt wird, in dem sich das Übertragungsmittel befindet und nach der Übertragung der Flüssigkeit auf das medizinische Implantat aus dem Behälter herausgezogen wird, und
das Übertragungsmittel porös und elastisch ist und als Kegelmantel oder als plane Scheibe oder als gewölbte Scheibe ausgebildet ist, wobei mindestens ein Einschnitt die Oberseite des Übertragungsmittels mit der Unterseite des Übertragungsmittels verbindet und das Implantat durch diesen mindestens einen Einschnitt in die Vorrichtung eingebracht wird.

2. Verfahren nach Anspruch 1, **dadurch gekennzeichnet, dass**
die Flüssigkeit aus dem Übertragungsmittel heraus auf die zu beschichtende Oberfläche des medizinischen Implantats übertragen wird, vorzugsweise aus Kanälen, zumindest einer Vorratskammer, Poren, Fasern und/oder Zwischenräumen des Übertragungsmittels.

3. Verfahren nach einem der Ansprüche 1 oder 2, **dadurch gekennzeichnet, dass**
die Flüssigkeit in das Übertragungsmittel eingebracht wird, vorzugsweise in Kanäle, zumindest eine Vorratskammer, Poren und/oder Zwischenräume des Übertragungsmittels, oder das Übertragungsmittel mit der Flüssigkeit getränkt wird, insbesondere unmittelbar vor der Verwendung.

4. Verfahren nach Anspruch 3, **dadurch gekennzeichnet, dass**
eine Wirkstofflösung oder Wirkstoffsuspension mit einem oder mehreren pharmazeutischen Wirkstoffen in das Übertragungsmittel eingebracht wird, wobei die Wirkstoffe aus der Gruppe ausgewählt sind, die aus Antibiotika, organischen Antiseptika, Kupfersalzen, Kupferoxid, Galliumsalzen, Strontiumsalze, Lthiumsalzen, Silbersalzen, Silberoxid, Bisphosphonaten, Wachstumsfaktoren, Steroidhormonen, nichtsteroidalen Hormonen, Hämostyptika, Antiphlogistika, Plasmiden, Cosmiden, linearer DNA besteht.

5. Verfahren nach einem der vorangehenden Ansprüche, **dadurch gekennzeichnet, dass** nach dem Übertragen der Flüssigkeit auf das medizinische Implantat ein Pulver auf die benetzte Oberfläche des medizinischen Implantats aufgebracht wird, bevorzugt das medizinische Implantat in ein Pulver eingetaucht wird, wobei das Pulver zumindest eine pharmazeutisch aktive Substanz umfasst.

6. Verfahren nach einem der vorangehenden Ansprüche, **dadurch gekennzeichnet, dass** ein Teil der übertragenen Flüssigkeit und/oder des Pulvers abgestreift wird, insbesondere beim Herausziehen des medizinischen Implantats aus dem Behälter, vorzugsweise an einem dafür vorgesehenen Abstreifer.

7. Verfahren nach einem der vorangehenden Ansprüche, **dadurch gekennzeichnet, dass** die Flüssigkeit eingefärbt wird, so dass der beschichtete Bereich des medizinischen Implantats farblich kenntlich gemacht wird.

8. Verfahren nach Anspruch 7, **dadurch gekennzeichnet, dass**
die Vollständigkeit der Beschichtung des zu beschichtenden Bereichs anhand der Färbung geprüft wird.

9. Vorrichtung (1, 11) zur zumindest bereichsweisen Beschichtung eines medizinischen Implantats mit einem Verfahren nach einem der vorangehenden Ansprüche, wobei die Vorrichtung (1, 11) ein elastisch deformierbares Übertragungsmittel (8) umfasst, das eine Flüssigkeit aufweist, die zumindest eine pharmazeutisch aktive Substanz umfasst, so dass beim Kontaktieren des Übertragungsmittels (8) mit der zu beschichtenden Oberfläche des medizinischen Implantats die Flüssigkeit auf das medizinische Implantat übertragbar ist,
**dadurch gekennzeichnet, dass**
das Übertragungsmittel (8) in einem Behälter (4, 14) umfassend eine Öffnung zum Einführen und Herausnehmen des medizinischen Implantats angeordnet ist und
das Übertragungsmittel porös und elastisch ist und als Kegelmantel oder als plane Scheibe oder als gewölbte Scheibe ausgebildet ist, wobei mindestens ein Einschnitt die Oberseite des Übertragungsmittels mit der Unterseite des Übertragungsmittels verbindet.

10. Vorrichtung (1, 11) nach Anspruch 9, **dadurch gekennzeichnet, dass** die Öffnung durch einen abziehbaren Deckel verschlossen ist.

11. Vorrichtung (1, 11) nach einem der Ansprüche 9 oder 10, **dadurch gekennzeichnet, dass** die Vorrichtung (1, 11) einen Abstreifer (6, 16) umfasst, der vorzugsweise im Bereich der Öffnung, insbesondere zwischen der Öffnung und dem Übertragungsmittel (8), angeordnet ist.

12. Vorrichtung (1, 11) nach einem der Ansprüche 9 bis 11, **dadurch gekennzeichnet, dass** der Abstreifer (6, 16) scheibenförmig ist und mindestens einen Einschnitt (17) umfasst, der die Oberseite und die Unterseite der Scheibe (6) verbindet.

13. Vorrichtung (1, 11) nach einem der Ansprüche 9 bis 11, **dadurch gekennzeichnet, dass** der Abstreifer (6, 16) als Kegelmantel oder als Halbkugelfläche ausgebildet ist, wobei die Kegelspitze oder die Halbkugel in Richtung des Übertragungsmittels (8) ausgerichtet ist und vorzugsweise der Kegel oder die Halbkugel mindestens einen Einschnitt (17) enthalten, der die Oberseite und die Unterseite des Abstreifers (6, 16) verbindet.

14. Vorrichtung (1, 11) nach einem der Ansprüche 9 bis 13, **dadurch gekennzeichnet, dass** das Übertragungsmittel (8) Poren umfasst und in den Poren des Übertragungsmittels (8) die Flüssigkeit, vorzugsweise in Form einer Lösung und/oder Suspension, enthalten ist.

15. Vorrichtung (1, 11) nach einem der Ansprüche 9 bis 14, **dadurch gekennzeichnet, dass** die pharmazeutisch aktive Substanz Antibiotika und/oder organische Antiseptika beinhaltet, so dass die zu erzeugende Beschichtung eine pharmazeutisch wirksame Dosis beinhaltet.

16. Vorrichtung (1, 11) nach einem der Ansprüche 9 bis 15, **dadurch gekennzeichnet, dass** die Vorrichtung (1, 11) einen Vakuumanschluss umfasst, der mit einer Vakuumquelle verbindbar ist und der vorzugsweise zwischen dem Abstreifer (6, 16) und dem Übertragungsmittel (8) angeordnet ist.

17. Vorrichtung (1, 11) nach einem der Ansprüche 9 bis 16, **dadurch gekennzeichnet, dass** das Übertragungsmittel (8) aus einem hydrophilen Material gefertigt ist und vorzugsweise zumindest ein anderes Teil, insbesondere der Behälter (4, 14) und/oder der Abstreifer (6, 16), aus einem hydrophoben Material gefertigt sind.

## Claims

1. Method for coating at least regions of a medical implant, whereby
a medical implant with a surface to be coated is provided and
the surface of the medical implant to be coated is contacted to an elastically deformable transfer means that comprises at least one liquid that contains at least one pharmaceutical agent, whereby, upon contacting the transfer means, the liquid is transferred from the transfer means to the surface of the medical implant to be coated,
**characterised in that**
before contact with the transfer means, the medical implant is introduced into a container, in which the transfer means is situated, and is pulled out of the container after the liquid is transferred to the medical implant, and
the transfer means is porous and elastic and is provided in the form of a cone jacket or as a planar disk or as an arched disk, whereby at least one notch connects the top side of the transfer means to the bottom side of the transfer means and the implant is introduced into the device through said at least one notch.

2. Method according to claim 1, **characterised in that**
the liquid is transferred to the surface of the medical implant to be coated from the transfer means, preferably from channels, at least one reservoir chamber, pores, fibres and/or intervening spaces of the transfer means.

3. Method according to any one of the claims 1 or 2, **characterised in that** the liquid is introduced into the transfer means, preferably into channels, at least one reservoir chamber, pores and/or intervening spaces of the transfer means, or **in that** the transfer means is soaked with the liquid, in particular right before use.

4. Method according to claim 3, **characterised in that**
an agent solution or agent suspension with one or more pharmaceutical agents is introduced into the transfer means, whereby the agents are selected from the group consisting of antibiotics, organic antiseptic agents, copper salts, copper oxide, gallium salts, strontium salts, lithium salts, silver salts, silver oxide, bisphosphonates, growth factors, steroid hormones, non-steroidal hormones, haemostatic agents, antiphlogistic agents, plasmids, cosmids, linear DNA.

5. Method according to any one of the preceding claims, **characterised in that** following the transfer of liquid to the medical implant, a powder is applied to the wetted surface of the medical implant, preferably the medical implant is immersed in a powder, whereby the powder comprises at least one pharmaceutical agent.

6. Method according to any one of the preceding claims, **characterised in that** a part of the transferred liquid and/or of the powder is stripped off, in particular while the medical implant is being pulled out of the container, preferably on a stripper intended for this purpose.

7. Method according to any one of the preceding claims, **characterised in that** the liquid is being dyed such that the coated region of the medical implant is made recognisable by colour.

8. Method according to claim 7, **characterised in that**
the completeness of the coating of the region to be coated is tested by means of the coloration.

9. Device (1, 11) for coating at least regions of a medical implant by means of a method according to any one of the preceding claims, whereby the device (1, 11) comprises an elastically deformable transfer means (8) that comprises a liquid that comprises at least one pharmaceutical agent such that the liquid can be transferred to the medical implant, when the transfer means (8) is contacted to the surface of the medical implant to be coated,
**characterised in that**
the transfer means (8) is arranged in a container (4, 14) comprising an opening for introduction and removal of the medical implant, and **in that** the transfer means is porous and elastic and is provided as cone jacket or as planar disk or as arched disk, whereby at least one notch connects the top side of the transfer means to the bottom side of the transfer means.

10. Device (1, 11) according to claim 9, **characterised in that** the opening is closed by a lid that can be pulled off.

11. Device (1, 11) according to either one of the claims 9 or 10, **characterised in that** the device (1, 11) comprises a wiper (6, 16), which preferably is arranged at the opening, in particular between the opening and the transfer means (8).

12. Device (1, 11) according to any one of the claims 9 to 11, **characterised in that** the wiper (6, 16) is disk-shaped and comprises at least one notch (17) that connects the top side to the bottom side of the disk (6).

13. Device (1, 11) according to any one of the claims 9 to 11, **characterised in that** the wiper (6, 16) is provided as cone jacket or as semi-spherical surface, whereby the cone tip or the semi-sphere is oriented in the direction of the transfer means (8) and the cone or the semi-sphere preferably contains at least one notch (17) that connects the top side to the bottom side of the wiper (6, 16).

14. Device (1, 11) according to any one of the claims 9 to 13, **characterised in that** the transfer means (8) comprises pores and **in that** the pores of the transfer means (8) contain the liquid, preferably in the form of a solution and/or suspension.

15. Device (1, 11) according to any one of the claims 9 to 14, **characterised in that** the pharmaceutical agent includes antibiotics and/or organic antiseptic agents such that the coating to be generated includes a pharmaceutically effective dose.

16. Device (1, 11) according to any one of the claims 9 to 15, **characterised in that** the device (1, 11) comprises a vacuum connector that can be connected to a vacuum source and which preferably is arranged between the wiper (6, 16) and the transfer means (8).

17. Device (1, 11) according to any one of the claims 9 to 16, **characterised in that** the transfer means (8) are manufactured from a hydrophilic material and preferably, at least one other part, in particular the container (4, 14) and/or the wiper (6, 16), is/are manufactured from a hydrophobic material.

## Revendications

1. Procédé d'enduction au moins par région d'un implant médical, dans lequel un implant médical avec une surface à enduire est mis à disposition et
la surface à enduire de l'implant médical est mise en contact avec un moyen de transfert déformable élastiquement qui présente au moins un fluide qui contient au moins une substance pharmaceutiquement active, dans lequel le fluide est transféré du moyen de transfert sur la surface à enduire de l'implant médical lors de la mise en contact du moyen de transfert,
**caractérisé en ce que**
l'implant médical est introduit dans un récipient dans lequel le moyen de transfert se trouve avant le contact avec le moyen de transfert et est extrait du récipient après le transfert du fluide sur l'implant médical, et
le moyen de transfert est poreux et élastique, et est réalisé en tant qu'enveloppe conique ou en tant que disque plan ou en tant que disque bombé, dans lequel au moins une entaille connecte le côté supérieur du moyen de transfert au côté inférieur du moyen de transfert et l'implant est introduit dans le dispositif à travers cette au moins une entaille.

2. Procédé selon la revendication 1, **caractérisé en ce que**
le fluide est transféré du moyen de transfert sur la surface à enduire de l'implant médical, de préférence à partir de canaux, au moins d'une réserve, de pores, fibres et/ou espaces intermédiaires du moyen de transfert.

3. Procédé selon une des revendications 1 ou 2, **caractérisé en ce que**
le fluide est introduit dans le moyen de transfert, de préférence dans des canaux, au moins une réserve, des pores et/ou espaces intermédiaires du moyen de transfert, ou le moyen de transfert est imbibé du fluide, notamment directement avant l'utilisation.

4. Procédé selon la revendication 3, **caractérisé en ce que**
une solution d'ingrédients actifs ou suspension d'ingrédients actifs avec un ou plusieurs ingrédients actifs pharmaceutiques est introduite dans le moyen de transfert, dans lequel les ingrédients actifs sont sélectionnés parmi le groupe qui se compose d'antibiotiques, d'antiseptiques organiques, de sels de cuivre, de l'oxyde de cuivre, de sels de gallium, de sels de strontium, de sels de lithium, de sels d'argent, de l'oxyde d'argent, de bisphosphonates, de facteurs de croissance, d'hormones stéroïdiennes, d'hormones non stéroïdiennes, d'antihémorragiques, d'anti-inflammatoires, de plasmides, de cosmides, d'ADN linéaire.

5. Procédé selon une des revendications précédentes, **caractérisé en ce que** une poudre est appliquée sur la surface réticulée de l'implant médical après le transfert du fluide sur l'implant médical, de préférence l'implant médical est immergé dans une poudre, dans lequel la poudre comprend au moins une substance pharmaceutiquement active.

6. Procédé selon une des revendications précédentes, **caractérisé en ce que** une partie du fluide transféré et/ou de la poudre est raclée, notamment lors de l'extraction de l'implant médical du récipient, de préférence sur un racloir prévu à cet effet.

7. Procédé selon une des revendications précédentes, **caractérisé en ce que** le fluide est coloré de sorte que la région enduite de l'implant médical est identifiée de manière colorée.

8. Procédé selon la revendication 7, **caractérisé en ce que**
l'intégralité de l'enduction de la région à enduire est vérifiée à l'aide de la coloration.

9. Dispositif (1, 11) d'enduction au moins par région d'un implant médical avec un procédé selon une des revendications précédentes, dans lequel le dispositif (1, 11) comprend un moyen de transfert déformable élastiquement (8) qui présente un fluide qui comprend au moins une substance pharmaceutiquement active de sorte que lors de la mise en contact du moyen de transfert (8) avec la surface à enduire de l'implant médical, le fluide peut être transféré sur l'implant médical,
**caractérisé en ce que**
le moyen de transfert (8) est disposé dans un récipient (4, 14) comprenant une ouverture pour l'introduction et le retrait de l'implant médical, et
le moyen de transfert est poreux et élastique, et est réalisé en tant qu'enveloppe conique ou en tant que disque plan ou en tant que disque bombé, dans lequel au moins une entaille connecte le côté supérieur du moyen de transfert au côté inférieur du moyen de transfert.

10. Dispositif (1, 11) selon la revendication 9, **caractérisé en ce que**
l'ouverture est fermée par un couvercle amovible.

11. Dispositif (1, 11) selon une des revendications 9 ou 10, **caractérisé en ce que** le dispositif (1, 11) comprend un racloir (6, 16) qui est de préférence disposé dans la région de l'ouverture, notamment entre l'ouverture et le moyen de transfert (8).

12. Dispositif (1, 11) selon une des revendications 9 à 11, **caractérisé en ce que** le racloir (6, 16) est en forme de disque et comprend au moins une entaille (17) qui connecte le côté supérieur et le côté inférieur du disque (6).

13. Dispositif (1, 11) selon une des revendications 9 à 11, **caractérisé en ce que** le racloir (6, 16) est réalisé en tant qu'enveloppe conique ou en tant que surface hémisphérique, dans lequel la pointe conique ou l'hémisphère est orientée en direction du moyen de transfert (8) et le cône ou l'hémisphère contient de préférence au moins une entaille (17) qui connecte le côté supérieur et le côté inférieur du racloir (6, 16).

14. Dispositif (1, 11) selon une des revendications 9 à 13, **caractérisé en ce que** le moyen de transfert (8) comprend des pores et le fluide est contenu dans les pores du moyen de transfert (8), de préférence sous forme d'une solution et/ou suspension.

15. Dispositif (1, 11) selon une des revendications 9 à 14, **caractérisé en ce que** la substance pharmaceutiquement active contient des antibiotiques et/ou antiseptiques organiques de sorte que l'enduction à réaliser contient une dose pharmaceutiquement efficace.

16. Dispositif (1, 11) selon une des revendications 9 à 15, **caractérisé en ce que** le dispositif (1, 11) comprend un raccord à vide qui peut être connecté à une source de vide et qui est de préférence disposé entre le racloir (6, 16) et le moyen de transfert (8).

17. Dispositif (1, 11) selon une des revendications 9 à 16, **caractérisé en ce que** le moyen de transfert (8) est fabriqué à partir d'un matériau hydrophile et au moins une autre partie, notamment le récipient (4, 14) et/ou le racloir (6, 16), est de préférence fabriquée à partir d'un matériau hydrophobe.
